Europäisches Patentamt

European Patent Office

Office européen des brevets

(11) Veröffentlichungsnummer : **0 192 612**
**B1**

(12) **EUROPÄISCHE PATENTSCHRIFT**

(45) Veröffentlichungstag der Patentschrift :
25.05.88

(51) Int. Cl.⁴ : **C 07 C 85/24, C 07 C 87/60, C 07 C 76/02, C 07 C 79/32**

(21) Anmeldenummer : 86810088.4

(22) Anmeldetag : 17.02.86

(54) Verfahren zur Herstellung von Chlornitroanilinen und Chlornitrophenolen.

(30) Priorität : 22.02.85 CH 827/85

(43) Veröffentlichungstag der Anmeldung :
27.08.86 Patentblatt 86/35

(45) Bekanntmachung des Hinweises auf die Patenterteilung : 25.05.88 Patentblatt 88/21

(84) Benannte Vertragsstaaten :
BE CH DE FR GB LI

(56) Entgegenhaltungen :
EP-A- 0 041 908
EP-A- 0 161 650
DE-C-  432 801
CHEMICAL ABSTRACTS, Band 51, Nr. 7, 10. April 1957, Nr. 5008e, Columbus, Ohio, US; L.M. LITVI-NENKO et al.: "Synthesis of some halogen-contai-ning amino and nitro derivatives of biphenyl"
CHEMICAL ABSTRACTS, Band 90, Nr. 15, 9. April 1979, Seite 605, Nr. 121176d, Columbus, Ohio, US
CHEMICAL ABSTRACTS, Band 101, Nr. 9, 27. August 1984, Seite 614, Nr. 72408r, Columbus, Ohio, US
CHEMICAL ABSTRACTS, Band 103, Nr. 15, 14. Okto-ber 1985, Seite 694, Nr. 123139e, Columbus, Ohio, US

(73) Patentinhaber : CIBA-GEIGY AG
Klybeckstrasse 141
CH-4002 Basel (CH)

(72) Erfinder : Albrecht, Bernhard
Im Laig 173
CH-4463 Buus (CH)
Erfinder : Beyrich, Jürgen, Dr.
Am Tischliweg 3
D-7859 Huttingen (DE)

**Beschreibung**

Die Erfindung betrifft ein Verfahren zur Herstellung von Chlornitroanilinen und Chlornitrophenolen durch Chlorieren der entsprechenden Nitroaniline oder Nitrophenole in wässriger Salzsäure als Reaktionsmedium.

Chlorierte Nitroaniline und Nitrophenole sind wichtige Zwischenprodukte für die Farbstoffsynthese und dienen hier insbesondere zur Herstellung von Dispersionsfarbstoffen. In der Literatur sind zahlreiche Verfahren zur Herstellung dieser Verbindungen beschrieben. Zu unterscheiden ist zwischen der direkten Chlorierung geeigneter Ausgangsverbindungen und der Aminolyse oder Hydrolyse entsprechender Nitro-chlorderivate.

Mittels Chlorierung erhält man beispielsweise das 2-Chlor-4,6-dinitroanilin durch Umsetzen von 2,4-Dinitroanilin mit Kaliumchlorat in Salzsäure (P. G. van de Vliet ; Rec. Trav. Chim. 43 610 [1924]) oder mit elementarem Chlor in Wasser, in Gegenwart von Eisen-III-chlorid (DE-C-610613), ferner mit trockenem Chlor in Eisessig in Gegenwart von Antimon (CS-A-152,603). Von diesen Chlorierungsmethoden hat die Verfahrensvariante mit Chlorat in Salzsäure breite Anwendung gefunden. Danach wird das 2,4-Dinitroanilin in Salzsäure vorgelegt und das Chlorat als wässrige Lösung langsam zugegeben. Nachteil dieser Verfahrensführung ist jedoch, dass das Ausgangsmaterial nur unvollständig zum gewünschten Endprodukt umgesetzt wird. Versucht man den Umsatz durch eine längere Reaktionszeit zu verbessern, so wird die Reaktionsmasse dickflüssig und es kommt zu einem stossweise Aufschäumen unter Entweichen von Chlorgas. Diese Methode befriedigt daher weder hinsichtlich der Ausbeute noch der Verfahrenssicherheit.

Auch die Chlorierung von 2,4-Dinitrophenol mit elementarem Chlor in konzentrierter Salzsäure brachte bisher keine befriedigenden Ergebnisse. Beobachtet wird die Bildung chlorierter Nebenprodukte durch den Austausch von Nitrogruppen gegen Chloratome (EP-A-124 084).

Aufgabe der vorliegenden Erfindung ist es daher, ein Verfahren zu entwickeln, wonach sich Nitroaniline und Nitrophenole möglichst vollständig chlorieren lassen, und das zudem einfach und sicher in der Durchführung ist.

Gefunden wurde, dass sich ein praktisch vollständiger Umsatz des Ausgangsmaterials, bei maximaler Verfahrenssicherheit auf einfache Weise dadurch erreichen lässt, dass man die als Reaktionsmedium dienende Salzsäure vorlegt und getrennte Ströme von Nitroanilin oder Nitrophenol und Chlorierungsmittel gleichzeitig zudosiert. Durch diese Verfahrensweise ist sichergestellt, dass das Ausgangsmaterial sehr rasch, d. h. kurz nach dem Eintragen in die Vorlage, chloriert wird. Dadurch kommt es zu keiner Anhäufung von Chlorierungsmittel im Reaktionsgemisch, wodurch ein plötzliches Aufschäumen der Reaktionsmasse und die Bildung überchlorierter Produkte weitgehend vermieden wird.

Gegenstand der vorliegenden Erfindung ist somit ein Verfahren zur Herstellung von Chlornitroanilinen oder Chlornitrophenolen, durch Chlorieren der entsprechenden Nitroaniline oder Nitrophenole in Salzsäure, das dadurch gekennzeichnet ist, dass man die Salzsäure vorlegt und getrennte Ströme von Nitroanilin oder Nitrophenol und Chlorierungsmittel gleichzeitig zudosiert.

Als Ausgangsmaterial für das erfindungsgemässe Verfahren kommen in erster Linie Mono- oder Dinitroaniline oder Mono- oder Dinitrophenole in Betracht. Genannt sind das ortho-, meta- oder para-Nitroanilin, das 2,4-Dinitroanilin, 2,6-Dinitroanilin, 3,4-Dinitroanilin, 3,5-Dinitroanilin ; das ortho ; meta- oder para-Nitrophenol, das 2,4-Dinitrophenol, 2,6-Dinitrophenol, 3,4-Dinitrophenol sowie das 3,5-Dinitrophenol. Neben der Nitrogruppe können die Nitroaniline oder -phenole noch weitere, in der Chlorierung inerte Substituenten aufweisen. Gute Ergebnisse werden im vorliegenden Verfahren insbesondere mit dem 2,4-Dinitroanilin, sowie dem p-Nitrophenol erhalten. Diese Verbindungen lassen sich mit sehr guten Ausbeuten zu den in 6- oder 2,6-Stellung chlorierten Verbindungen umsetzen.

Die genannten Ausgangsverbindungen sind bekannt oder nach bekannten Methoden zugänglich, so z. B. das 2,4-Dinitroanilin durch Erhitzen von 4-Chlor-1,3-dinitrobenzol in wässrigem Ammoniak unter Zusatz von Chinolin (US 2 072 618). Eine Uebersicht betreffend Verfahren zur Herstellung von Nitrophenolen findet sich in Ullmanns Encyklopädie der technischen Chemie 3. Auf 1. Bd. 13, Seiten 5 ff (1962).

Bei der als Reaktionsmedium verwendeten Salzsäure handelt es sich allgemein um 0,5 bis 37 Gew.%ige, zweckmässigerweise um 10 bis 35 Gew.%ige Salzsäure. Dabei verwendet man auf 1 Teil Edukt im allgemeinen 2 bis 15 Teile Salzsäure.

Als Chlorierungsmittel verwendet man in erster Linie elementares Chlor oder Alkalichlorate, z. B. Natrium- oder Kaliumchlorat, zweckmässigerweise in Form wässriger oder salzsaurer Lösungen. Daneben kommen auch Alkalihypochlorite, wie z. B. Natriumhypochlorit in Betracht, das ebenfalls als wässrige Lösung eingesetzt wird. Ferner können Chlorierungskatalysatoren, wie Jod oder Uebergangsmetallchloride, wie z. B. Eisen-III-chlorid verwendet werden.

Eingesetzt wird das Chlorierungsmittel zweckmässigerweise in etwa äquimolarer Menge, je einzuführendes Chloratom mit einem Ueberschuss von 1 bis 50 Gew.%, bevorzugt arbeitet man mit einem Ueberschuss von 10 bis 20 Gew.%. Man kann auch mit weniger als der äquimolaren Menge an Chlorierungsmittel auskommen, wenn man dem Reaktionsgemisch eine entsprechende Menge an Wasserstoffperoxid zusetzt.

Durchgeführt wird die Chlorierung vorteilhaft bei einer Temperatur von —10 °C bis 80 °C, insbesondere bei einer Temperatur von 0° bis 40 °C oder 5 bis 40 °C. Reaktionstemperaturen über 80 °C führen zu einer vermehrten Bildung an Nebenprodukten, während die Chlorierung bei Temperaturen unter —10 °C zu langsam verläuft. Ferner sollte beim Arbeiten in konzentrierter Salzsäure eine Reaktionstemperatur von 40 °C nicht überschritten werden, da sonst, je nach Ausgangsverbindung, grössere Mengen an Nebenprodukten entstehen.

Das als Ausgangsverbindung verwendete Nitroanilin oder Nitrophenol wird zweckmässigerweise in Form einer wässrigen oder salzsauren Suspension oder Anschlämmung in die vorgelegte Salzsäure eingetragen. Aufgrund der geringen Löslichkeit der Edukte in der Salzsäure erweist es sich als vorteilhaft, diese mittels einer Nassmahlung in eine feindisperse Form zu überführen. Durch diese Massnahme erreicht man eine deutliche Erhöhung der Reaktionsgeschwindigkeit und damit der Raum-Zeit-Ausbeute. Zur Nassmahlung der wässrigen Anschlämmung verwendet man die üblichen Mahlaggregate, wie z. B. Rührwerks-Kugelmühlen, Sandmühlen oder Stiftscheibenmühlen. Ferner kann zur Zerkleinerung des Ausgangsmaterials auch Ultraschall verwendet werden. Je nach angestrebtem Feinheitsgrad der Suspension beträgt die Mahldauer 1 bis 5 Stunden. Um zu einer möglichst stabilen Dispersion zu gelangen, kann man die Mahlung in Gegenwart eines Dispergiermittels durchführen. Als Dispergiermittel kommen in erster Linie anionische und nicht-ionische oberflächenaktive Substanzen in Betracht, beispielsweise Aethylenoxidaddukte von Fettalkoholen, Alkylphenolen, Fettaminen oder Fettsäuren, die gegebenenfalls verestert sind, ferner Formaldehydkondensate aromatischer Sulfonsäuren, Ligninsulfonate oder Oxyligninsulfonate. Das Dispergiermittel wird üblicherweise in einer Menge von 0,1 bis 5 Gew.%, insbesondere 0,2 bis 1 Gew.%, bezogen auf Edukt dem Mahlslurry beigegeben.

Es ist vielfach ausreichend, wenn der kristalline Anteil des Edukts auf eine mittlere Teilchengrösse von kleiner als 100 μm gemahlen wird.

Der Verdünnungsgrad der Eduktströme und die Menge an vorgelegter Salzsäure wird so bemessen, dass die Produktkonzentration in der Reaktionsmasse am Ende der Reaktion zweckmässigerweise 5 bis 30 Gew.%, insbesondere 10 bis 20 Gew.% beträgt. In der Regel ist ein Produktgehalt von mehr als 30 Gew.% wegen der zunehmend schlechter werdenden Rührbarkeit des Reaktionsgemisches zu vermeiden.

Zur Aufarbeitung kann man das Reaktionsgemisch zunächst mit einem Inertgas, z. B. Stickstoff durchspülen und auf diese Weise überschüssiges Chlor entfernen. Letzte Chlorreste können gegebenenfalls noch mittels Hydrogensulfit zerstört werden. Anschliessend isoliert man das im Reaktionsmedium unlösliche Produkt mit Hilfe üblicher Trennmethoden, wie z. B. durch Filtrieren, Dekantieren oder Zentrifugieren. Vorteilhaft filtriert man das Produkt ab, wäscht es gegebenenfalls neutral und trocknet es anschliessend. Die als Filtrat zurückbleibende Salzsäure kann direkt im nachfolgenden Ansatz wieder vorgelegt und erneut als Reaktionsmedium verwendet werden. Das erfindungsgemässe Verfahren kann diskontinuierlich aber auch kontinuierlich durchgeführt werden. Für kontinuierliche Verfahren eignen sich die bekannten Vorrichtungen und Apparaturen, wie z. B. Durchflussrührkessel, Rührkesselkaskade oder auch ein Schlaufenreaktor. Gegebenenfalls wird das Reaktionsgemisch zur Vervollständigung der Reaktion noch in einen Nachreaktor geleitet, beispielsweise ein Strömungsrohr oder eine Rührkesselkaskade oder einfach in einen Nachreaktionskessel. Die Verweilzeit des Reaktionsgemischs im Reaktor ist abhängig von der Reaktivität des jeweiligen Edukts und der Reaktionstemperatur.

Das erfindungsgemässe Verfahren hat gegenüber bekannten Verfahren, wonach das zu chlorierende Edukt im Reaktionsmedium vorgelegt und anschliessend das Chlorierungsmittel zugegeben wird, die folgenden Vorteile :

praktisch vollständiger Umsatz des Ausgangsmaterials ; das Produkt hat einen Reinheitsgrad von grösser als 98 % und kann direkt in der Farbstoffsynthese verwendet werden ;

kurze Reaktionszeit und damit verbunden eine höhere Raum-Zeit-Ausbeute ;

kein Zusatz an Tensiden nötig, da die Reaktionsmasse wenn überhaupt, nur geringfügig schäumt ;

keine Akkumulation an Chlorat und damit auch kein Aufschäumen des Reaktionsgemisches durch plötzlich freigesetztes Chlor ;

bessere Kristallform des Produkts und damit verbunden eine leichtere Abtrennbarkeit, insbesondere Filtrierbarkeit ;

problemlose Wiederverwendung der gebrauchten Salzsäure ist möglich ;

kein Viskositätsanstieg während der Reaktion, eine gleichbleibend gute Rührbarkeit des Reaktionsgemisches ist gewährleistet.

Das erfindungsgemässe Chlorierungsverfahren kann im einzelnen beispielsweise folgendermassen durchgeführt werden : Der Nitroaromat z. B. 2,4-Dinitroanilin wird in etwa der gleichen Gewichtsmenge Wasser angeschlämmt und gemahlen. Als Dispergator wird gegebenenfalls ein Ligninsulfonat zugegeben. Im Reaktor legt man ca. 15 Gew.%ige Salzsäure vor ; es kann hier die Salzsäure aus dem vorhergehenden Ansatz verwendet werden. Auf 1 Teil 2,4-Dinitroanilin verwendet man 4 bis 5 Teile Salzsäure. Anschliessend dosiert man die feingemahlene 2,4-Dinitroanilin-Anschlämmung und elementares Chlor bei Raumtemperatur, (20 bis 25 °C) simultan innerhalb von 4 bis 5 Stunden in die vorgelegte Salzsäure. Anstelle bei Raumtemperatur kann man auch bei 5 °C oder 40 °C arbeiten.

Ist die gesamte Menge an 2,4-Dinitroanilin und die entsprechende Menge an Chlorgas — letzteres in äquimolarer Menge und einem etwa 10 %igen Ueberschuss — in die Salzsäure eingetragen lässt man noch eine halbe bis eine Stunde nachreagieren und bläst dann überschüssiges Chlor mittels Stickstoff aus. Anschliessend filtriert man das Produkt ab, wäscht neutral und trocknet. Man erhält das 2-Chlor-4,6-dinitroanilin nach diesem Verfahren in einer Ausbeute von etwa 95 % mit einem Gehalt an nicht umgesetztem Ausgangsmaterial (Dinitroanilin) von weniger als 0,5 Gew.%.

Die nach dem erfindungsgemässen Verfahren hergestellten Chlornitroaniline und Chlornitrophenole dienen vor allem zur Herstellung von Dispersionsfarbstoffen, die insbesondere zum Färben hydrophober Fasermaterialien, wie z. B. solche aus Polyestern geeignet sind.

Die folgenden Beispiele dienen der Veranschaulichung der Erfindung ; Teile bedeuten Gewichtsteile und Prozente Gewichtsprozente.

## Beispiel 1

183 Teile 2,4-Dinitroanilin werden in 220 Teilen Wasser angeschlämmt und gemahlen. Im Reaktor legt man 800 Teile 14,2 %ige Salzsäure vor. Die Salzsäure kann aus einem vorhergehenden Ansatz stammen und wird nach Abtrennen des Produkts wieder in den Reaktor zurückgeführt. In die Salzsäure dosiert man bei einer Temperatur von 25 °C simultan die wässrige Dinitroanilin-Suspension und insgesamt 78 Teile Chlor ein, wobei die Zugabe der Dinitroanilin-Suspension innerhalb von 240 Minuten erfolgt, während das Chlor noch 20 Minuten länger, also insgesamt 260 Minuten eingeleitet wird. Anschliessend lässt man das Reaktionsgemisch noch 30 Minuten nachreagieren und bläst während 30 Minuten überschüssiges Chlor aus. Dann filtriert man das Produkt ab, wäscht neutral und trocknet. Man erhält 204 Teile 2-Chlor-4,6-dinitroanilin, das entspricht einer Ausbeute von 94 %. Schmelzpunkt : 157,5 °C (Lit. : 157-159 °C). In der gaschromatographischen Analyse zeigt das Produkt die gleiche Retentionszeit, wie authentisches Material. Führt man die Reaktion bei 40 °C durch, so erreicht man analoge Ergebnisse.

## Beispiel 2

183 Teile 2,4-Dinitroanilin werden zusammen mit 0,5 Teilen Ligninsulfonat in 220 Teilen Wasser angeschlämmt und gemahlen. Im Reaktor legt man 800 Teile 32 %ige Salzsäure vor. In die Salzsäure dosiert man bei einer Temperatur von 25 °C gleichzeitig in getrennten Strömen 145 Teile einer 33 %igen Natriumchloratlösung und die durch Nassmahlung erhaltene wässrige Dinitroanilin-Suspension. Die Chloratlösung wird innerhalb von 260 Minuten und gleichzeitig damit die Dinitroanilin-Suspension in einem etwas kürzeren Zeitraum von 240 Minuten zugegeben. Anschliessend lässt man das Gemisch noch 30 Minuten nachreagieren, bläst überschüssiges Chlor aus und zerstört die im Gemisch zurückbleibenden Chlorreste durch Zusetzen einer Natriumhydrogensulfitlösung. Danach filtriert man das Produkt ab, wäscht neutral und trocknet. Man erhält 204 Teile 2-Chlor-4,6-dinitroanilin (Ausbeute 94 %) ; Schmelzpunkt : 157,5 °C (Lit. : 157-159 °C).

## Beispiel 3

139 Teile p-Nitrophenol werden in 200 Teilen Wasser angeschlämmt und gemahlen. Die so erhaltene feinteilige p-Nitrophenol-Suspension wird anschliessend gleichzeitig mit 260 Teilen einer 33 %igen Natriumchloratlösung in 1250 Teilen 33 %ige Salzsäure eindosiert. Die gleichzeitige Zugabe von Edukt und Chlorierungsmittel erfolgt bei einer Temperatur von 25 °C, wobei das p-Nitrophenol innerhalb von 240 Minuten zugegeben wird, während die Zugabe des Natriumchlorats 20 Minuten länger innerhalb von 260 Minuten erfolgt. Anschliessend lässt man noch 30 Minuten nachreagieren und bläst innerhalb einer weiteren halben Stunde Chlorrückstände aus. Zurückbleibendes Chlor wird mittels Natriumhydrogensulfit zerstört. Schliesslich filtriert man das Produkt ab wäscht neutral und trocknet. Man erhält 199,5 Teile 2,6-Dichlor-4-nitrophenol in einer Reinheit von 99,7 %. Die Ausbeute beträgt 95,6 %; Schmelzpunkt : 119-120 °C (Lit. : 125 °C). In der gaschromatographischen Analyse zeigt das Produkt die gleiche Retentionszeit, wie authentisches Material.

Analoge Ergebnisse erhält man, wenn man die Reaktion bei 40 °C durchführt.

## Beispiel 4

139 Teile p-Nitrophenol werden in 292 Teilen Wasser angeschlämmt und gemahlen. Im Reaktor legt man 1250 Teile 20 %ige Salzsäure vor, die bereits in einem vorhergehenden Ansatz als Reaktionsmedium diente und nach Abtrennen des Produkts in den Reaktor zurückgeführt wird. Zur vorgelegten Salzsäure dosiert man bei einer Temperatur von 25 °C simultan 156 Teile Chlor und die p-Nitrophenol-Suspension ; die Nitrophenolsuspension wird innerhalb von 240 Minuten und gleichzeitig damit, das Chlor innerhalb von 260 Minuten, also 20 Minuten länger zugegeben. Anschliessend lässt man 30 Minuten nachreagieren und bläst innerhalb von weiteren 30 Minuten überschüssiges Chlor aus. Das Produkt wird abfiltriert, neutralgewaschen und getrocknet. Man erhält 200 Teile 2,6-Dichlor-4-nitrophenol, das entspricht einer Ausbeute von 96 % ; Schmelzpunkt : 119-120 °C (Lit. : 125 °C).

## Beispiel 5

160 Teile 2,4-Dinitrophenol werden in 240 Teilen Wasser angeschlämmt. Die Anschlämmung wird mit 0,5 Teilen Ligninsulfonat (Sulfitablauge) versetzt und das Dinitrophenol durch Beschallen mit Ultraschall zerkleinert.

Im Reaktor legt man 210 Teile 14 %ige Salzsäure vor und dosiert bei einer Temperatur von 0 °C simultan die wässrige Suspension des feingemahlenen 2,4-Dinitrophenols und 60 Teile Chlorgas ein, wobei die Zugabe der Dinitrophenol-Suspension innerhalb von 120 Minuten erfolgt, während das Chlor gleichzeitig damit über einen Zeitraum von 130 Minuten zugegeben wird. Nach beendeter Chlorzugabe wird das Produkt abfiltriert und mit 250 Teilen Wasser gewaschen. Man erhält 162,4 Teile 2-Chlor-4,6-dinitrophenol, das entspricht einer Ausbeute von ca. 83 %.

## Patentansprüche

1. Verfahren zur Herstellung von Chlornitroanilinen oder Chlornitrophenolen durch Chlorieren von Nitroanilinen oder Nitrophenolen in Salzsäure, dadurch gekennzeichnet, dass man die Salzsäure vorlegt und getrennte Ströme von Nitroanilin oder Nitrophenol und Chlorierungsmittel gleichzeitig zudosiert.

2. Verfahren nach Anspruch 1, dadurch gekennzeichnet, dass man als Ausgangsmaterial Mono- oder Dinitroaniline oder Mono- oder Dinitrophenole chloriert.

3. Verfahren nach Anspruch 2, dadurch gekennzeichnet, dass man 2,4-Dinitroanilin oder p-Nitrophenol chloriert.

4. Verfahren nach Anspruch 1, dadurch gekennzeichnet, dass man Salzsäure einer Konzentration von 10 bis 35 Gew.% vorlegt.

5. Verfahren nach Anspruch 1, dadurch gekennzeichnet, dass das Chlorierungsmittel elementares Chlor oder ein Alkalichlorat ist.

6. Verfahren nach Anspruch 1, dadurch gekennzeichnet, dass man das Chlorierungsmittel in äquimolarer Menge je einzuführendes Chloratom mit einem Ueberschuss von 1 bis 50 Gew.% einsetzt.

7. Verfahren nach Anspruch 6, dadurch gekennzeichnet, dass man das Chlorierungsmittel mit einem 10 bis 20 Gew.%igen Ueberschuss, bezogen auf die äquimolare Menge je einzuführendes Chloratom, einsetzt.

8. Verfahren gemäss Anspruch 1, dadurch gekennzeichnet, dass man die Reaktion bei einer Temperatur von —10° bis 80 °C durchführt.

9. Verfahren nach Anspruch 8, dadurch gekennzeichnet, dass man die Reaktion bei einer Temperatur von 0° bis 40 °C durchführt.

10. Verwendung der nach Anspruch 1 erhaltenen Chlornitroaniline und Chlornitrophenole zur Herstellung von Dispersionsfarbstoffen.

## Claims

1. A process for the preparation of a chloronitroaniline or chloronitrophenol by chlorinating a nitroaniline or nitrophenol in hydrochloric acid, which process comprises initially introducing the hydrochloric acid and simultaneously metering in separate streams of a nitroaniline or nitrophenol and of a chlorinating agent.

2. A process according to claim 1, which comprises chlorinating a mono- or dinitroaniline or a mono- or dinitrophenol as starting material.

3. A process according to claim 2, which comprises chlorinating 2,4-dinitroaniline or p-nitrophenol.

4. A process according to claim 1, which comprises initially introducing hydrochloric acid having a concentration of 10 to 35 % by weight.

5. A process according to claim 1, wherein the chlorinating agent is elemental chlorine or an alkali metal chlorate.

6. A process according to claim 1, wherein an excess of 1 to 50 % by weight of chlorinating agent, based on the equimolar amount per chlorine atom to be introduced, is used.

7. A process according to claim 6, wherein an excess of 10 to 20 % by weight of chlorinating agent, based on the equimolar amount per chlorine atom to be introduced, is used.

8. A process according to claim 1, wherein the reaction is carried out at a temperature from —10° to 80 °C.

9. A process according to claim 8, wherein the reaction is carried out at a temperature from 0° to 40 °C.

10. The use of a chloronitroaniline or chloronitrophenol obtained according to claim 1 for the preparation of a disperse dye.

## Revendications

1. Procédé pour la préparation de chloronitroanilines ou de chloronitrophénols, par chloration de nitroanilines ou de nitrophénols dans de l'acide chlorhydrique, caractérisé en ce qu'on ajoute simultanément, dans de l'acide chlorhydrique, des courants, distincts, de nitroaniline ou de nitrophénol et d'un agent de chloration.

2. Procédé selon la revendication 1, caractérisé en ce qu'on chlore, en tant que substances de départ, des mono- ou dinitroanilines ou des mono- ou dinitrophénols.

3. Procédé selon la revendication 2, caractérisé en ce qu'on chlore la 2,4-dinitroaniline ou le p-nitrophénol.

4. Procédé selon la revendication 1, caractérisé en ce qu'on emploie de l'acide chlorhydrique ayant une concentration de 10 à 35 % en poids.

5. Procédé selon la revendication 1, caractérisé en ce que l'agent de chloration est le chlore élémentaire ou un chlorate de métal alcalin.

6. Procédé selon la revendication 1, caractérisé en ce qu'on utilise l'agent de chloration avec un excès de 1 à 50 % en poids par rapport à la quantité équimolaire correspondant aux atomes de chlore à introduire.

7. Procédé selon la revendication 6, caractérisé en ce qu'on utilise l'agent de chloration avec un excès de 10 à 20 % en poids par rapport à la quantité équimolaire correspondant aux atomes de chlore à introduire.

8. Procédé selon la revendication 1, caractérisé en ce qu'on met en oeuvre la réaction à une température de —10 à 80 °C.

9. Procédé selon la revendication 8, caractérisé en ce qu'on met en oeuvre la réaction à une température de 0 à 40 °C.

10. Utilisation des chloronitroanilines et des chloronitrophénols obtenus selon la revendication 1, pour la préparation de colorants de dispersion.